# EUROPEAN PATENT APPLICATION

(11) **EP 1 849 435 A2**
(43) Date of publication of application: **31.10.2007**
(21) Application number: 07014230.2
(22) Date of filing: 22.03.2004
(51) Int. Cl.: A61F 2/06

(54) **Stent with attached sleeve marker**

(30) Priority: 16.06.2003 US 463114
(62) Divisional of application: 04251635.1
(71) Applicant: Endotex Interventional Systems, Inc., Cupertino, CA 95014 (US)
(72) Inventor: Yang, Yi, San Francisco California 94116 (US); Ong, Ich, San Jose California 95111 (US); Hogben, Scott, San Mateo California 94403 (US)
(74) Representative: McLeish, Nicholas Alistair Maxwell

(57) **Abstract**

A stent for endovascular placement in the body having radiopaque markers in the shape of sleeves attached to struts of the stent. In a preferred embodiment, radiopaque sleeve markers are attached surrounding the terminal end of struts at the ends of a stent so that the location within the body of the stent can be identified by fluoroscopy or other methods. Upon placement of the sleeve marker on the stent strut, the marker is attached to the strut by a snap-fit mechanism, or by deforming or changing the shape of the marker and/or the strut to inhibit removal of the marker.

## Description

### FIELD OF THE INVENTION

This invention relates to prostheses devices for implantation within body lumens, generally called stents, and more particularly to stents with attached radiopaque markers.

### BACKGROUND OF THE INVENTION

Stents are generally tubular-shaped prostheses which are deployed in a blood vessel or other anatomical lumen. Stents are useful in the treatment of atherosclerotic stenosis in blood vessels, and are used to widen and circumferentially support and hold open a blood vessel that has been narrowed and occluded by vascular disease. These endoprostheses or stents generally consist of an expandable tubular member which has as small profile in a contracted state so that it can be delivered to the site by minimally invasive procedures such as percutenous transluminal angioplasty. Stents are also used in graft prostheses which are implanted within blood vessels to prevent vessel wall rupture, particularly in the aorta or other arteries which may be subject to aneurysm formation and/or severe atherosclerotic disease which may involve multiple stenoses.

A variety of structures are used as stents or intraluminal vascular grafts, including stents made of wire mesh or slotted tubular elements, coiled springs, helical wire coil, and coiled sheet and helical mesh stents such as, e.g., those described in U.S. Patent Nos. 5,824,054 and 6,425,915, among others. Stents are typically loaded into a catheter in a contracted state and percutaneously introduced and advanced to a treatment site within a blood vessel, and may be balloon-expandable or self-expanding.

In order to precisely place a stent at the desired location, it is necessary to identify the exact position of the stent within a blood vessel. One means used to accomplish this is to incorporate a radiopaque marker into the stent so that, through the use of fluoroscopy, the position of the stent within the blood vessel can be identified, both at the time of placement and during subsequent checkups. For example, U.S. Pat. No. 6,409,752 describes a stent having a waveform pattern formed from a flat sheet of material in which a radiopaque marker of gold, platinum, tungsten, or iridium is positioned in a circular eyelet opening formed through the thickness of the sheet at the ends of the stent. The marker is positioned in the eyelet by melting the marker material in place or by crimping or other fastening methods. U.S. Pat. No. 6,402,777 describes radiopaque markers for use in stents formed by compressing, welding, or fusing a rivet of gold, tantalum or platinum through an opening in the ends or edges of the stent. Other methods for enabling the precise identification of a stent location include coating or plating the stent edges as markers as described in, e.g., European Patent Application No. 95302708 and U.S. Pat. No. 6,086,604; filling hollow stent wire with radiopaque material; producing the stent itself from radiopaque material such as tantalum; and using thicker metal at the ends of the stent as described in US2002/0072792A1.

Given the importance of precise placement of a stent at the desired location within a blood vessel, there is a need for additional approaches to providing stents with markers to aid in achieving proper stent placement for a variety of stent designs.

### SUMMARY OF THE INVENTION

According to a first aspect of the present invention, there is provided an endovascular stent having a radiopaque marker comprising: a stent of biocompatible material which is formed into struts surrounding open areas, the stent having at least one elongated terminating strut, the at least one terminating strut having a proximal end and a distal end, the at least one terminating strut connected to the rest of the stent at only the proximal end, the at least one terminating strut having a first side and a second side, the first side and the second side each having a proximal region with a proximal cross-sectional dimension and a distal region with a distal cross-sectional dimension, wherein the proximal cross-sectional dimension is less than the distal cross-sectional dimension; and a marker in the shape of a sleeve and formed of material which is more radiopaque than the material forming the stent, said marker being placed on and surrounding a terminating strut of the stent about the proximal regions of the first side and second side, and wherein said marker is attached to the stent.

According to a second aspect of the present invention, there is provided aa method of attaching a radiopaque marker to a stent, comprising: providing a stent formed of struts surrounding open areas, the stent having at least one elongated terminating strut, the at least one elongated terminating strut having a proximal end and a distal end the at least one elongated terminating strut having a first side and a second side, the first side and the second side each having a proximal region with a proximal cross-sectional dimension and a distal region with a distal cross-sectional dimension, wherein the proximal cross-sectional dimension is less than the distal cross-sectional dimension; providing a marker in the shape of a sleeve, the marker being made of a material that is more radiopaque than the material forming the stent; sliding the marker over the distal end of the at least one terminating strut and advancing the marker to a position surrounding the terminating strut at the proximal regions of the first side and second side; and attaching the marker to the at least one terminating strut to thereby inhibit the marker from sliding off the distal end of the strut.

According to this invention, a marker in the shape of an open-ended sleeve is formed from radiopaque material, such as a platinum or tantalum, for attachment to a strut at the edges of an endoprostheses or stent. The sleeve marker is slid over and around the terminating end of the stent strut and the marker is permanently secured to the strut by changing the shape of the marker or the strut or by providing a snap-fit mechanism for retaining the marker on the strut. For example, where the strut is provided with an indentation or slot opening through the strut, the marker may be crimped and permanently deformed so that a portion of the marker extends below the surface of the stent strut. Alternately, the sides of a slotted strut may be expanded to widen the profile of the strut distal to the marker to prevent subsequent removal of the marker from the strut. The profile of the terminal end of the strut may also be expanded or increased in size after the sleeve marker is placed on the strut by other ways, including by forming a weld ball in the end of the strut having a sufficient diameter to prevent the marker from being removed from the strut. Preferably, the terminal end of the strut is provided with a portion having an increased width that is adapted to contract slightly and then snap back into place as the sleeve marker slides over it, thereby retaining the sleeve marker in place on the terminal strut.

Sleeve markers according to this invention can be used with any type of stent or stent graft so long as the stent has strut portions over which the sleeve marker can be slid and secured into place. The marker can be formed of a variety of materials which are more radiopaque than the material forming the stent and which can be identified by fluoroscopy or other such techniques.

There is provided an endovascular stent having a radiopaque marker comprising: a stent of biocompatible material which is formed into struts surrounding open areas, said stent having one or more elongated terminating struts connected to the rest of the stent at only one end of said terminating strut; a marker in the shape of a sleeve and formed of material which is more radiopaque than the material forming the stent, said marker being placed on and surrounding a terminating strut of the stent, and wherein said marker is attached to the stent.

Advantageously, the terminating strut has a slot through a portion of the strut extending longitudinally along the length of the strut but not extending to the terminal end of the stent. More advantageously, the sides of the strut surrounding the slot have been permanently deformed to extend outward distal to the marker to an extent sufficient to thereby prevent removal of the marker from the strut.

Advantageously, the terminal end of the strut distal to the marker has a cross-sectional dimension that is larger than a cross-sectional dimension of the marker by an amount sufficient to inhibit removal of the marker from the strut.

Advantageously, the marker is attached to the stent by a snap-fit mechanism. More advantageously, said snap-fit mechanism includes a pocket located on a portion of said terminating strut, said pocket adapted to retain the marker thereon. Still more advantageously, said pocket is defined by a differential in the cross-sectional dimension of at least two portions of the terminating strut. Yet more advantageously, said terminating strut has a distal section and a narrowed section, said distal section having a cross-sectional dimension that is larger than said narrowed section. More advantageously still, said marker has an internal cross-sectional dimension that is smaller than the cross-sectional dimension of the distal section of said terminating strut.

There is also provided a method of attaching a radiopaque marker to a stent, comprising: providing a stent formed of struts surrounding open areas, said stent and having one or more elongated terminating struts; providing a marker in the shape of a sleeve, said marker being made of a material that is more radiopaque than the material forming the stent; and sliding the marker over the terminal end of the terminating strut and advancing the marker to a position surrounding the terminating strut.

Advantageously, the method further comprises attaching the marker to the terminating strut to thereby inhibit the marker from sliding off the terminal end of the strut.

Advantageously, the terminating strut has a snap-fit mechanism for attaching the marker to the terminating strut to thereby inhibit the marker from sliding off the terminal end of the strut. More advantageously, said snap-fit mechanism includes a pocket located on a portion of said terminating strut, said pocket adapted to retain the marker thereon, said pocket being defined by a differential in the cross-sectional dimension of at least two portions of the terminating strut, and wherein said terminating strut has a distal section and a narrowed section, said distal section having a cross-sectional dimension that is larger than said narrowed section, and wherein said marker has a internal cross-sectional dimension that is smaller than the cross-sectional dimension of the distal section of said terminating strut.

### BRIEF DESCRIPTION OF THE DRAWINGS

FIG. 1 shows a preferred illustrative embodiment of a mesh sheet for a stent of the present invention having terminating struts at its ends.
FIG. 2 shows an enlarged view of a portion of a terminating stent strut which has a slot along its length.
FIG. 3 shows a sleeve marker of the present invention prior to its attachment to the stent strut.
FIG. 4 shows a sleeve marker placed surrounding a slotted stent strut and crimped on one side into the slot of the strut.
FIG. 5 shows the sleeve marker crimped on two opposite sides into the slot of the stent strut.
FIG. 6 shows an alternate embodiment in which the sides of the slot of the stent strut have been expanded outward distal to a sleeve marker placed around the strut.
FIG. 7 shows a terminating slotted stent strut having a stop member against which a sleeve marker can be placed.
FIG. 8 shows a sleeve marker placed surrounding the terminating slotted stent strut of FIG. 7 with the sides of the slot expanded outward distal to the sleeve marker.
FIGS. 9a-9c show an alternate embodiment of a sleeve marker attached to a stent.
FIG. 10 shows a still further alternate embodiment of a sleeve marker attached to a stent.

### DETAILED DESCRIPTION OF THE PREFERRED EMBODIMENTS

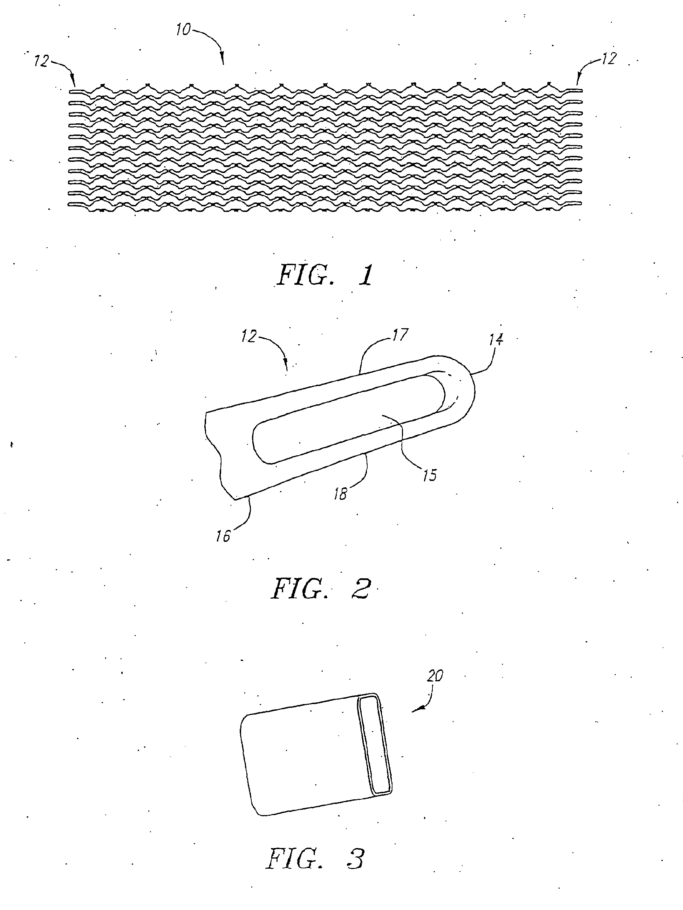

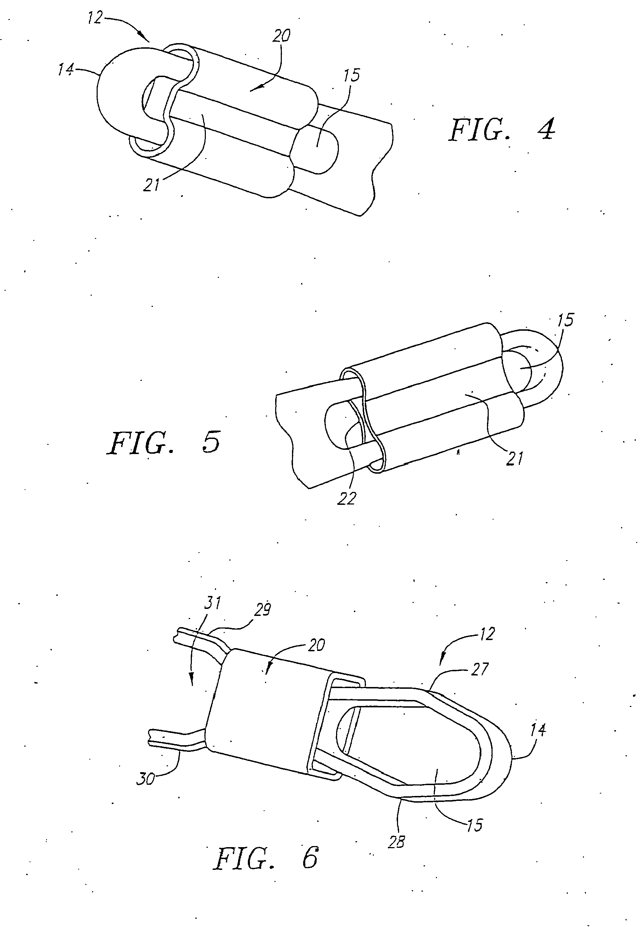

FIG. 1 shows a preferred illustrative embodiment of a stent **10** having terminating struts **12** at its edges to which sleeve markers can be attached in accordance with the present invention. As shown in FIG. 1, the terminating stent struts **12** are elongated portions which are formed as part of the stent, but are connected to the other portions of the stent only at one end and have a free terminal end. In a particularly preferred embodiment, the stent is a mesh coiled sheet stent formed of a mesh sheet as shown in FIG. 1 (shown prior to being coiled into the tube shape of the stent), having struts forming open expandable cells and having terminating struts at each end of the stent. Such a stent is similar to those described in United States Patent Application S/N 10/462876, entitled "Coiled-Sheet Stent With Flexible Mesh Design," assigned to Endotex Interventional Systems, Inc., and filed on the same date as the present application. The aforementioned patent application is hereby incorporated herein by reference in its entirety as if fully set forth herein. However, many other types of stents are also contemplated by this invention so long as the stent contains terminating struts to which sleeve markers can be attached. The stent and struts may be made of any suitable material, including a shape memory material such as Nitinol.

FIG. 2 shows an enlargement of an illustrative terminating stent strut **12.** FIG. 2 shows the free terminal end **14** of the strut and the opposite end **16** by which the strut is attached to the rest of the stent. Stent strut **12** shown in FIG. 2 is a slotted strut which has an elongated slot **15** formed in the strut between the strut's terminal end **14** and its attachment end **16.** The strut **12** has strut sides **17** and **18** parallel to the slot **15.** As shown in the embodiment shown in FIG. 2, slot **15** extends entirely through the strut. As one example of such a slotted strut, for a stent thickness of 0.0065 inches and a terminating strut measuring 0.012 inches wide and approximately 0.05 inches long, the slot **15** may be positioned toward the free end of the strut **12** and measure 0.006 inches wide and 0.034 inches long. As will be apparent to those of skill in the art, many other sizes and dimensions for a stent strut and/or a strut slot will also be appropriate. Instead of a slot, the strut can alternately have one or more indentations along the side surfaces of the strut which do not extend entirely through the strut.

FIG. 3 shows a preferred embodiment of a sleeve marker **20** prior to attachment to a stent strut. The marker **20** is formed in the shape of a hollow tube or sleeve shown, which is open at both ends as shown. The marker is formed from a radiopaque metal such as platinum, gold, tantalum, tungsten, iridium, etc., which is more radiopaque than that of the material from which the stent is formed. In a preferred embodiment, the material from which the marker is made is tantalum. The sleeve marker **20** is of a size and formed into a shape corresponding generally to that of the stent strut, and which can be slipped over and around the terminal end **14** of a strut **12** of the stent. For a strut having a generally rectangular cross-section such as the example described above, the sleeve marker will preferably also have a generally rectangular cross-section to match that of the strut. If the metal from which the sleeve marker 20 is to be formed is provided in round tube form, the marker tube material can be shaped to more closely fit the generally rectangular cross-section of the strut by, for example, choosing a mandrel of appropriate size and shape and forcing the marker tubing over the mandrel to form it into the desired rectangular shape.

A marker **20** of appropriate size and shape relative to the stent strut **12** is provided and slid over and around a terminating stent strut **12,** and is centered over the slot **15.** The marker **20** is then permanently attached to the strut **12** by crimping, and specifically by using force to permanently deform the marker **20** so that part of the marker is pushed into the slot **15.** For example, the stent strut **12** may be placed in a holder with a forming die sized and shaped to firmly hold the strut. An aluminum dowel with a flat tip may be used to push the sides of the marker **20** into the fixture, deforming the marker and pushing part of it into the slot **15** on the strut **12.** As shown in FIG. 4, the resulting "crimped" side portion **21** of the marker extends below the surface level of the strut **12** into the slot **15,** thereby providing positive attachment of the marker **20** to the strut by physically preventing the marker from sliding off the terminal end **14** of the strut due to its crimped shape. The marker can also be crimped on both sides, as shown in FIG. 5, by compressing and permanently deforming both of opposite sides **21** and **22** of the sleeve marker **20** down into the slot **15** and below the surface of the strut **12.** This results in a tighter fit of the sleeve marker **20** to the strut **12,** and provides a further means of preventing the marker from dislodging off the end of the strut. For a stent strut having one or more indentations, rather than a slot through the strut, the marker is similarly crimped into the indention(s) and is thereby permanently deformed to achieve positive attachment of the sleeve marker to the stent strut.

Alternately, a sleeve marker may be attached to a slotted stent strut formed of a shape memory material such as Nitinol by taking advantage of those shape memory properties. As shown in FIG. 6, in this embodiment the marker **20** is placed over and around a slotted terminating stent strut **12,** and the marker is pushed onto the strut past most or all of the slot **15** in strut **12** until the marker contacts a wider area of the stent adjacent to or formed by struts **29** and **30** of the adjoining open cell **31** of the stent. An expansion mandrel (not shown) is inserted into the slot **15** in the stent strut, resulting in outwardly deformed sides **27** and **28** of the strut. As an example, a 0.010 inch mandrel may be used for the exemplary sleeve marker and slotted strut of the size described above. The stent slot is then heat-treated and the mandrel removed from the strut slot, resulting in a permanent deformation and widening of the strut slot which prevents the marker **20** from being slipped off the terminating strut. For example, the minimum outside dimension of the outwardly deformed sides **27** and **28** of the strut is greater than the maximum inside dimension of the marker **20.** This method of attaching the sleeve marker **20** to the stent strut has the advantage that the marker material is subjected to a reduced amount of forming during the process, since only the initial forming of the sleeve marker into the corresponding shape of the strut is required.

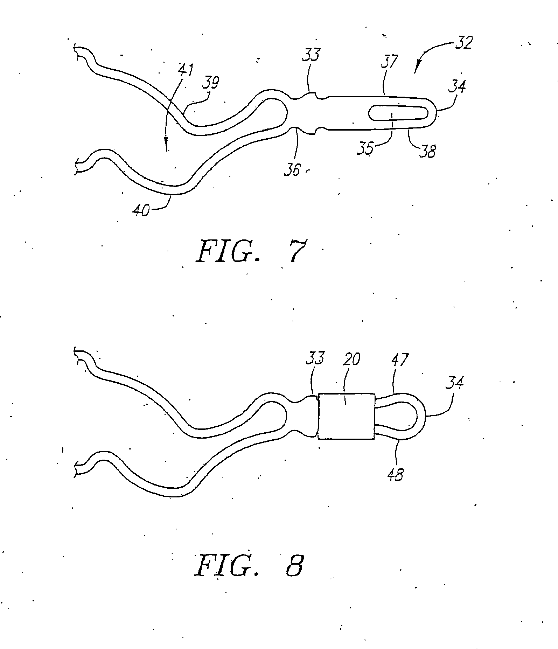

FIG. 7 shows a preferred embodiment of a slotted terminating stent strut **32** which has a free terminal end **34,** and stop member **33** at the opposite end **36** by which the strut is attached to the rest of the stent by struts **39** and **40** of the first open cell **41.** Sleeve marker **20** is pushed onto the stent strut **32** until the marker contacts stop member **33,** and then an expansion mandrel is forced into the slot **35** of strut **32** distal to marker **20,** resulting in outwardly deformed sides **47** and **48** of the strut and thereby widening of the strut slot distal to the marker, as shown in FIG. 8. The stent strut is then heat-treated, as discussed above, resulting in permanent deformation and widening of the strut slot and preventing removal of marker **20.**

The sleeve markers of this invention are preferably attached to struts at the ends of a stent, but could also be attached to any position along the stent having a terminating strut. Sleeve markers may be attached to multiple terminating struts of a stent. As an example, multiple sleeve markers may be attached to terminating struts evenly spaced around the entire periphery at each end of a stent, and in a preferred embodiment the sleeve markers are attached to alternating terminating struts. In a particularly preferred embodiment, five sleeve markers are attached to alternating terminating struts at each end of a mesh coil stent formed from the stent material shown in FIG 1. The marker material is more radiopaque than that of the stent. Therefore, after the stent with attached sleeve markers is loaded into a catheter and placed within a lumen in the body, the exact location of the stent, indicated by the attached markers, can be shown by fluoroscopy or other such techniques.

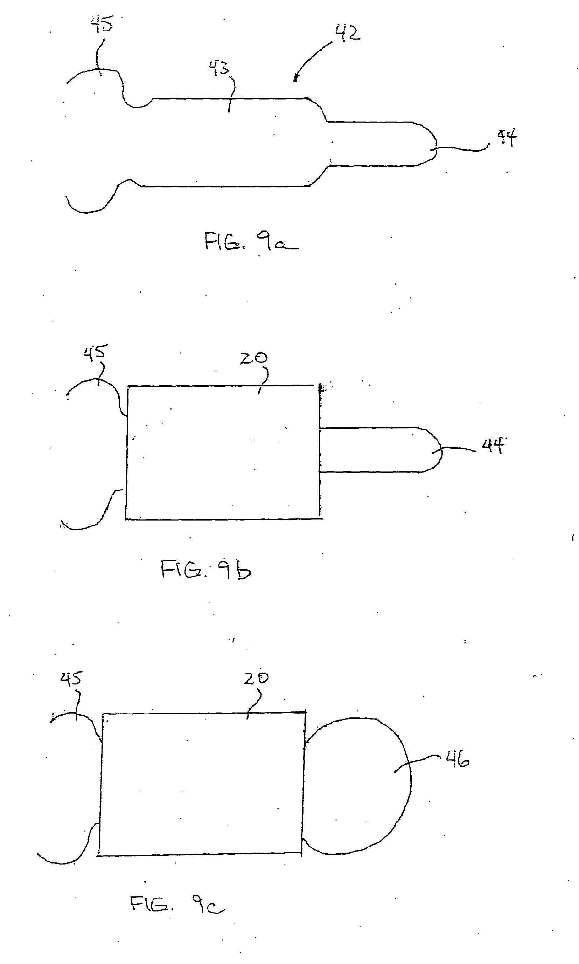

In an alternative embodiment, the sleeve marker is held in place on a terminating strut of a stent by other means of increasing the size of the terminal end of the strut distal to the sleeve marker after the marker has been slipped onto the strut. The terminating stent struts need not be slotted in this embodiment. For example, as shown in FIGS. 9a-9c, a weld ball may be formed on the end of the strut, the weld ball having a sufficient diameter to prevent removal of the sleeve marker from the stent strut. FIG. 9a shows a terminating strut **42** having a support portion **43,** an end **44,** and a stop member **45.** The support portion **43** has a width adapted to retain a sleeve marker **20,** and the stop member **45** has a size sufficient to prevent axial movement of the sleeve marker beyond the point of the stop member. Turning to FIG. 9b, a sleeve marker **20** is placed over the support portion to be held on the terminating strut. Once it is located, the end **44** of the terminating strut is melted to form a ball **46** having an outside diameter larger than the internal diameter of the sleeve marker, to thereby hold the sleeve marker in place, as shown in FIG. 9c. Melting of the end **44** of the terminating strut is preferably achieved by exposing the end material to a laser, although other methods are known to those of skill in the art. Further, although a ball-shape is preferred, the strut end **44** may be melted to form any shape capable of retaining the sleeve marker on the terminating strut.

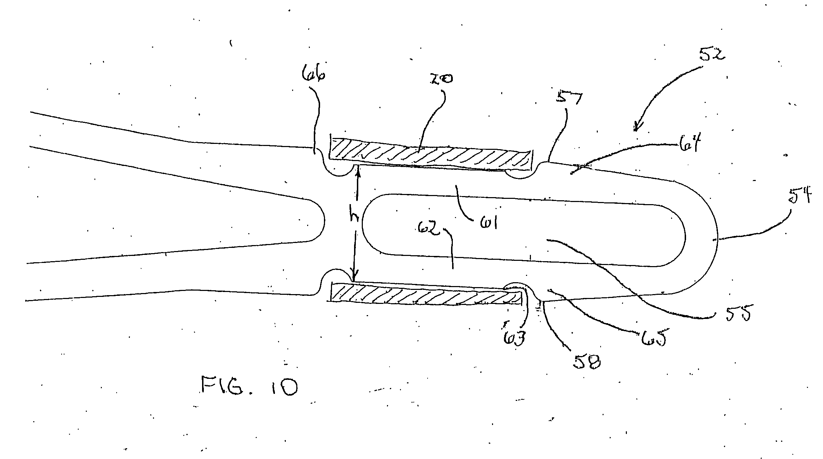

In the preferred embodiment shown in FIG. 10, a sleeve marker **20** (shown in cross-section) is held in place on a terminating stent strut **52** by forming the terminating strut having a width that cooperates to lock the sleeve marker **20** in a pocket **63** once it is placed over the terminating strut **52.** As shown in FIG. 10, the strut sides **57** and **58** are provided with narrowed sections **61** and **62,** the first narrowed section **61** being formed on the upper side of the upper strut side **57,** and the second narrowed section **62** being formed on the lower side of the lower strut side **58.** The narrowed sections **61** and **62** are formed by slightly thinning the exterior portion of the strut sides **57** and **58** at the same longitudinal location near the end **54** of the terminating strut to create a pocket **63** within which the sleeve marker **20** is located. The distance **h** between the upper edge of the first narrowed section **61** and the lower edge of the second narrowed section **62** generally corresponds to the inner dimension of the sleeve marker so that the sleeve marker fits snugly over the narrowed sections when the sleeve marker is placed on the terminating strut. The distal sections **64** and **65** of the terminating strut are not thinned, with the result that the width of the terminating strut **52** is slightly larger at the location of the distal sections **64** and **65** as compared to the width of the terminating strut at the location of the narrowed sections **61** and **62,** thereby creating the pocket **63** within which the sleeve marker is located. The sleeve marker **20** is placed on the terminating stent strut **52** by sliding it over the end **54** until the sleeve marker engages the narrowed sections **61** and **62** and is located in the pocket **63.** As the sleeve marker passes over the distal sections **64** and **65,** which have a slightly larger width than the internal dimension of the sleeve marker, the sleeve marker causes the distal sections **64** and **65** to compress slightly into the space formed by the slot **55,** thereby allowing the sleeve marker to pass over the distal sections **64** and **65** until coming to rest in the pocket **63.** After the sleeve marker passes over the distal sections **64** and **65,** the distal sections snap back into place, locking the sleeve marker in place. A ledge **66** is formed on the stent where the terminating strut **52** connects to the stent body. The ledge **66** acts as a stop to prevent movement of the sleeve marker past the location of the ledge **66.**

A variety of other methods can also be used to increase one or more of the dimensions of the terminal end of the strut distal to the sleeve marker by an amount sufficient to prevent removal of a marker placed onto the strut, or to provide a snap-fit mechanism on the distal end of the terminating strut, as will be apparent to those of skill in the art. For example, compression forces can be applied to the terminal end of the strut distal to the sleeve marker to permanently deform the strut's terminal end such that its width is increased beyond the width of the marker.

While preferred illustrative embodiments of the invention are described above, it will be apparent to one skilled in the art that various changes and modifications may be made thereto without departing from the invention, and which are accordingly within the scope of the invention taught herein.

## Claims

1. An endovascular stent having a radiopaque marker comprising:
a stent of biocompatible material which is formed into struts surrounding open areas, the stent having at least one elongated terminating strut, the at least one terminating strut having a proximal end and a distal end, the at least one terminating strut connected to the rest of the stent at only the proximal end, the at least one terminating strut having a first side and a second side, the first side and the second side each having a proximal region with a proximal cross-sectional dimension and a distal region with a distal cross-sectional dimension, wherein the proximal cross-sectional dimension is less than the distal cross-sectional dimension; and
a marker in the shape of a sleeve and formed of material which is more radiopaque than the material forming the stent, said marker being placed on and surrounding a terminating strut of the stent about the proximal regions of the first side and second side, and wherein said marker is attached to the stent.

2. The stent of claim 1, further comprising at least one proximal ledge, the at least one proximal ledge being formed adjacent to where the at least one terminating strut is connected to the rest of the stent, the at least one proximal ledge having a cross-sectional dimension greater than the proximal cross-sectional dimension such that the proximal ledge acts as a stop to prevent movement of the marker past the location of the at least one proximal ledge.

3. The stent of claim 1, further comprising at least one distal ledge, the at least one distal ledge being formed between the proximal region and the distal region, the at least one distal ledge having a cross-sectional dimension greater than the proximal cross-sectional dimension such that the distal ledge acts as a stop to prevent movement of the marker past the location of the at least one distal ledge.

4. The stent of claim 1, wherein the terminating strut has a slot through a portion of the strut extending longitudinally along the length of the strut but not extending to the distal end of the strut.

5. The stent of claim 4, wherein the first side is a first stent member, the first stent member defining at least a portion of the slot, and wherein the second side is a second stent member, the second stent member being joined to the first stent member and defining at least a portion of the slot.

6. The stent of claim 5, wherein the second stent member is joined to the first stent member at the proximal end and at the distal end, and wherein the proximal end is integral with the rest of the stent.

7. The stent of claim 1, wherein the marker is attached to the stent by a snap-fit mechanism.

8. The stent of claim 7, wherein the snap-fit mechanism includes a pocket located on a portion of the at least one terminating strut, the pocket adapted to retain the marker thereon.

9. The stent of claim 8, wherein the pocket is defined by the proximal regions of the first side and second side.

10. A method of attaching a radiopaque marker to a stent, comprising:
providing a stent formed of struts surrounding open areas, the stent having at least one elongated terminating strut, the at least one elongated terminating strut having a proximal end and a distal end the at least one elongated terminating strut having a first side and a second side, the first side and the second side each having a proximal region with a proximal cross-sectional dimension and a distal region with a distal cross-sectional dimension, wherein the proximal cross-sectional dimension is less than the distal cross-sectional dimension;
providing a marker in the shape of a sleeve, the marker being made of a material that is more radiopaque than the material forming the stent;
sliding the marker over the distal end of the at least one terminating strut and advancing the marker to a position surrounding the terminating strut at the proximal regions of the first side and second side; and
attaching the marker to the at least one terminating strut to thereby inhibit the marker from sliding off the distal end of the strut.
